# EUROPÄISCHE PATENTSCHRIFT

(11) **EP 0 691 408 B1**
(45) Veröffentlichungstag und Bekanntmachung des Hinweises auf die Patenterteilung: **08.11.2000**
(21) Anmeldenummer: 95110563.4
(22) Anmeldetag: 06.07.1995
(51) Int. Cl.: C12Q 1/00, G01N 27/327, G01N 33/497

(54) **UV-polymerisierbare Enzympaste zur Herstellung von Biosensoren und damit hergestellte Biosensoren**
UV polymerizable enzyme paste for the production of biosensors and biosensors produced therewith
Pâte d'enzyme polymérisable par UV pour la production de capteurs biologiques et capteurs produits en utilisant cela

(30) Priorität: 08.07.1994 DE 4424179
(43) Veröffentlichungstag der Anmeldung: 10.01.1996
(73) Patentinhaber: Gesellschaft für Biotechnologische Forschung mbH (GBF), D-38124 Braunschweig (DE)
(72) Erfinder: Rohm, Ingrid,, D-38124 Braunschweig (DE); Bilitewski, Ursula, Dr., D-38124 Braunschweig (DE)
(74) Vertreter: Boeters, Hans Dietrich, Dr.

(56) Entgegenhaltungen:
- EP-A- 0 652 436
- CHEMICAL ABSTRACTS, Band 122, Nr. 25, 19. Juni 1995, Columbus, Ohio, USA I. ROHM et al. "UV-Polymeri- zable Screen-Printed Enzyme Pastes" Seite 466, Nr. 309 959v; & Anal. Chem. 1995, 67(13), 2304-7
- CHEMICAL ABSTRACTS, Band 101, Nr. 17, 22. Oktober 1984, Columbus, Ohio, USA E.A. YASTREBOVA "Blood alcohol determination using an enzyme paste electrode" Seite 176, Nr. 145 265d; & Deposited Doc. 1982, VINITI 3676-83, 147-50
- A. Riklin & I. Willner (1995) Anal. Chem. 67, 4118-4126.
- I. Rohm et al. (1995) Anal. Chem. 67, 2304-2307

## Beschreibung

Die vorliegende Erfindung betrifft eine UV-polymerisierbare Enzympaste zur Herstellung von Dickschicht-Biosensoren, insbesondere betrifft die vorliegende Erfindung eine siebdruckfähige UV-polymerisierbare Enzympaste, die die Massenproduktion von Biosensoren erlaubt sowie damit hergestellte Dickschicht-Biosensoren für amperometrische Meßverfahren.

Mit Hilfe der Dickschichttechnik erzeugte amperometrische Biosensoren für die medizinische Analytik befinden sich bereits seit einiger Zeit auf dem Markt, z.B. Medisense, Bayer (Mass Production of Biosensors, M. Alvarez-Icaza; Anal. Chem. 1993, 65, 525A-533A); auf den Inhalt dieser Schrift wird für die vorliegende Erfindung voll Bezug genommen.

Ein bisher unbefriedigendes Detail bei der industriellen Herstellung der Sensoren ist die reproduzierbare Aufbringung der analyt-spezifischen Enzyme auf die Oberfläche der Sensoren, die durch Applikation einer Enzymmembran, das Auftropfen oder Aufspritzen einer Enzymlösung erfolgt. Zur Lösung dieses Problems wurde die Verwendung von Pasten vorgeschlagen, die Enzyme, hauptsächlich H₂O₂ oder O₂ produzierende Oxidasen, enthalten, die im Siebdruckverfahren aufgebracht werden können. Dadurch wird die komplette Herstellung der Sensoren mit Hilfe einer einzigen Arbeitstechnik in industriellen Maßstab ermöglicht.

Die bislang bekannten siebdruckfähigen Enzympasten enthalten neben den Enzymen jeweils mindestens einen wasserlöslichen Vernetzer, wie Gelatine, Hydroxyethylcellulose, Cellulose-Derivate, Polyvinylalkohol oder Polyvinylpyrrolidon. Graphit dient zur Adsorption der Enzyme und als katalytisch wirksames Elektrodenmaterial zur Oxidation von H₂O₂ und O₂, durch die die Quantifizierung des Analyten erfolgt. Als weitere Zusätze finden Edelmetalle, z.B. Platin, und Mediatoren, z.B. Ferrocene, Kalium-Hexacyanoferrat und andere, Verwendung.

Diese Pasten, die eine wasserhaltige Matrix haben, werden auf die Elektrodenoberfläche aufgetragen, und die Aushärtung wird durch einfache Trocknung bei 4 °C über Nacht durchgeführt. Enzympasten und Dickschicht-Biosensoren, die unter Verwendung derartiger Pasten hergestellt worden sind, sind von Bilitewski et al. in "Miniaturized Disposable Biosensors", Sensors Actuators, B7 (1992) 351-355 und von Rüger in "Amperometrische Enzymsensoren auf der Basis der Dickschichttechnologie", Dissertation, TU-Braunschweig 1992, beschrieben worden, auf deren Inhalt für die vorliegende Erfindung voll Bezug genommen wird. Für Biosensoren vergleiche auch Chemnitius, Dissertation, 1994.

Jedoch können aufgrund der wasserhaltigen Pastenmatrix und der gleichzeitig großen Kornoberfläche des enthaltenen Graphit-Materials Schwierigkeiten bei dem Druck der Enzympaste auf die Oberfläche der Arbeitselektrode, insbesondere auf eine Platinoberfläche, wie folgt auftreten: Nachdem die Paste als dünne Schicht (kleiner 100 µm) im Druckprozeß auf dem Drucksieb von einem Druckrakel verteilt worden ist, beginnt eine relativ starke Verdunstung des Wassers aus der Paste, so daß diese von dem Druckrakel nicht gleichmäßig auf die Elektrodenoberfläche gepreßt wird, sondern teilweise - und bereits getrocknet - in den Maschen des Siebes zurückbleibt. Der Druckprozeß muß dann unterbrochen und das Sieb vor einer erneuten Benutzung gereinigt werden. Daher ist mit diesen Pasten ein kontinuierlicher Druckprozeß über einen längeren Zeitraum nicht möglich, und eine Massenproduktion von Dickschicht-Biosensoren erschwert.

Im Rahmen der Automatisierung bisher manuell ausgeführter Arbeitsabläufe werden Durchflußsysteme, wie die Fließinjektions-Analyse, verstärkt zur naßchemischen Detektion unterschiedlicher Parameter eingesetzt. Dickschichtsensoren haben sich in derartigen Systemen als geeignete Detektoren erwiesen. Der Einsatz von mit wasserlöslichen Enzympasten bedruckten Dickschicht-Elektroden in diesen relativ stark von einem Trägerstrom durchspülten Analysegeräten kann jedoch zur Auf- und Ablösung der Enzymschicht des Biosensors führen, wodurch sich die Größe des vom Sensor erzeugten Signals ändert und eine exakte Messung unmöglich wird.

Aus der Dünnschichttechnik sind Sensoren bekannt, die unter Verwendung UV-polymerisierbarer Materialien hergestellt werden (Rüger in "Amperometrische Enzymsensoren auf der Basis der Dickschichttechnologie", Dissertation, TU-Braunschweig 1992). In der Dünnschichttechnik werden - im Gegensatz zum Druckprozeß insbesondere in der Dickschichttechnik - die Sensorstrukturen durch Polymerisation aufgrund Belichtung durch Belichtungsschablonen erzeugt.

Aus Chemical Abstracts, Band 101, Seite 176 (1984), Abstract 145265d ist ein Verfahren zur Bestimmung von Blutalkohol unter Verwendung einer Alkohol-Dehydrogenase enthaltenden Enzympaste auf einer Elektrode offenbart.

In der EP 0 652 463 wird ein Biosensor mit einer Alkohol-Dehydrogenase enthaltenden Enzympaste auf einer Elektrode zum Bestimmen der Alkohol-Konzentration in der Atemluft offenbart.

Rohm et al. beschreiben in Anal. Chem. Band 67, Seiten 2304 - 2307 (1995) einen Dickschicht-Biosensoren auf der Grundlage einer siebdruckfähigen, Glukoseoxydase enthaltenden, UV-polymerisierbaren Paste.

Es ist Aufgabe der vorliegenden Erfindung, eine siebdruckfähige UV-polymerisierbare Enzympaste für die Herstellung von Dickschicht-Biosensoren in Massenproduktion zur Verfügung zu stellen, womit die Nachteile des Standes der Technik vermieden werden.

Diese Aufgabe wird durch eine UV-polymerisierbare Enzympaste zur Herstellung von Dickschicht-Biosensoren gelöst, die eine UV-polymerisierbare Siebdruckfarbe als Basismaterial, mindestens ein in das Basismaterial eingebrachtes Enzym und gegebenenfalls Mediatoren und/oder Co-Faktoren enthält.

Ferner können Enzymstabilisatoren enthalten sein.

Gegenstand der vorliegenden Erfindung ist auch ein mit dieser Enzympaste hergestellter Dickschicht-Biosensor, der für amperometrische Meßmethoden eingesetzt werden kann.

Vorteilhafte Ausführungsformen der Erfindung sind Gegenstand der Unteransprüche.

Mit der erfindungsgemäßen Enzympaste wird eine dauerhafte Sieboffenhaltung während des Druckprozesses gewährleistet, da die verwendeten Pastenmaterialien nur unter starkem UV-Licht auspolymerisieren und aufgrund ihrer chemischen Zusammensetzung nicht bei Raumtemperatur trocknen.

Weiter wird eine hohe mechanische Festigkeit der polymerisierten Immobilisierungsmatrix erhalten, wobei bei Dauertests keine Auswaschung des Enzyms beobachtet werden konnte, sondern im Gegensatz zu anderen kovalenten Immobilisierungen eine höhere Enzymstabilität beobachtet worden ist.

Die eigentliche Herstellung des amperometrischen Sensors und die Immobilisierung der Enzyme kann mittels einer Arbeitstechnik, des Siebdrucks, in großtechnischem Maßstab erfolgen.

Ein zusätzlicher Vorteil liegt darin, daß für alle eingesetzten nicht-enzymatischen Werkstoffe, wie Pastenmaterialien, Trägerstoffe, Siebdruckmaschinen und Belichtungsgeräte, herkömmliche Werkstoffe bzw. Mittel verwendet werden können, die routinemäßig und in großem Umfang in der Siebdruckindustrie eingesetzt werden, und daher jederzeit relativ preiswert kommerziell erhältlich sind.

Zudem kann der gesamte Immobilisierungsprozeß vom Mischen der Enzympaste bis zur Aushärtung innerhalb kürzester Zeit, z.B. in weniger als 3 Minuten, durchgeführt werden, da kein zeitraubender Trocknungsprozeß erforderlich ist.

Weiter sind mit UV-Siebdrucktechnik erzeugte Enzymschichten besser reproduzierbar als solche, die mittels herkömmlicher Glutaraldehyd-Immobilisierung hergestellt werden.

Für die erfindungsgemäße UV-polymerisierbare Enzympaste können herkömmliche, kommerziell erhältliche UV-polymerisierbare Basismaterialien verwendet werden, die speziell für den Siebdruck entwickelt worden sind und somit optimale physikalische Eigenschaften für die Anwendung besitzen, beispielsweise Siebdruckfarben.

In diese Matrix werden nach herkömmlichen bekannten Verfahren gelöste oder auch auf geeignete Trägermaterialien immobilisierte Enzyme eingebracht. Als Enzyme können bekannte Oxidasen, wie Glucoseoxidase, Lactatoxidase, Alkoholoxidase oder Pyranoseoxidase, sowie Dehydrogenasen, wie Alkohol-Dehydrogenase und Glucose-Dehydrogenase, oder Hydrolasen, wie Urease, Acetylcholinesterase oder Butyrylcholinesterase, verwendet werden.

Zudem können der erfindungsgemäßen UV-polymerisierbaren Enzympaste auch übliche aus der Literatur bekannte Zusätze, wie sie z.B. in Zusammenhang mit dem Stand der Technik beschrieben worden sind, zugesetzt werden.

Beispiele für Co-Faktoren sind Nikotinadenindinukleotide (NAD(P)).

Beispiele für Mediatoren sind Dimethylferrocen und Tetrathiafulvalen.

Beispiele für Enzymstabilisatoren sind die für konventionelle Enzymmembranen üblichen Stabilisatoren; vgl. beispielsweise Gibson et al. in Biosensors Bioelectronics, 7 (1992) 701-708; spezielle Beispiele sind bioverträgliche Polymere, Dextrane, Alkohole und Kohlehydrate.

Die erfindungsgemäße Enzympaste kann mittels herkömmlicher Technik und insbesondere Dickschichttechnik auf eine Elektrodenoberfläche, die z.B. aus Platin, Gold, Palladium und Legierungen zwischen diesen Materialien besteht, aufgebracht und polymerisiert werden.

Gemäß einer weiteren Ausführungsform betrifft die Erfindung einen Dickschicht-Biosensor, der durch eine auspolymerisierte UV-polymerisierbare Enzympaste gemäß der Erfindung gekennzeichnet ist.

Bei dem erfindungsgemäßen Biosensor kann die zu einer Enzymschicht auspolymerisierte Enzympaste eine Membran tragen, wobei diese Membran aus demselben Basismaterial mit denselben fakultativen Zusätzen wie die UV-polymerisierbare Enzympaste bestehen kann, jedoch keinen Gehalt an Enzymen aufweist.

Durch eine derartige Membran kann die Empfindlichkeit und der analytisch zugängliche Konzentrationsbereich variiert werden. Die Membran kann auf die Enzymschicht aufgebracht werden und wirkt als Diffusionsbarriere für den Analyten.

Bislang werden Lösungen geeigneter Polymere aufgepfropft, aufgesprüht oder durch spinn-coating aufgebracht, oder man taucht die Elektroden in die entsprechende Lösung ein. Die Membran bildet sich dann durch Verdampfen des Lösungsmittels. Dabei ist die Reproduzierbarkeit oft unbefriedigend. Die Reproduzierbarkeit fällt bei Verwendung einer fertigen Membran besser aus, eine derartige Membran muß aber mit Spezialvorrichtungen aufgeklebt werden.

Für die erfindungsgemäße Membran ist es vorteilhaft, daß sie genauso wie die Enzymschicht nach einem Siebdruckverfahren aufgebracht und danach durch UV-Belichtung ausgehärtet werden kann.
**Abb. 1** zeigt eine Glukose-Kalibrierkurve, die unter Verwendung einer erfindungsgemäßen UV-Enzym-Dickschichtelektrode (Dickschicht-Biosensor) und deren Einsatz in der Fließinjektions-Analyse erstellt worden ist.
**Abb. 2** zeigt das Ergebnis eines Dauertests, der mit einer erfindungsgemäßen UV-Enzym-Dickschichtelektrode (Dickschicht-Biosensor) bei Einsatz der Fließinjektions-Analytik mit 36 Injektionen einer 10 mM Glucoselösung pro Stunde und insgesamt 2880 Messungen durchgeführt worden ist.

### Beispiel

Im folgenden wird die Herstellung eines erfindungsgemäßen Dickschicht-Biosensors, UV-Enzym-Dickschichtelektrode, gemäß Rüger et al. in Sensors Actuators, B4 (1991) 267-271 Fig. 4 beschrieben.

Hierbei werden 50 mg UV-polymerisierbare Enzympaste (GL-396 Extender Base, Aquaglass GL; Sericol (Mühlheim)) mit 20 µl einer Glucoseoxidase-Lösung (30 mg/ml) gemischt, auf ein Sieb gegeben und durch Rakelbewegung auf die Oberfläche der Arbeitselektrode der herkömmlichen Dickschichtelektroden (Fabrikation in der GBF), die in diesem Fall aus Platin besteht, mit Hilfe einer Siebdruckmaschine in einer Schicht von etwa 50 bis 80 µm aufgedruckt.

Danach werden die Enzym-Elektroden zur Aushärtung der Enzym enthaltenden Schicht für 3 bis 5 Sekunden im Abstand von etwa 15 cm der Bestrahlung einer Mitteldruck-UV-Lampe bzw. eines herkömmlichen 80W-UV-Trockners mit integriertem Fließband ausgesetzt.

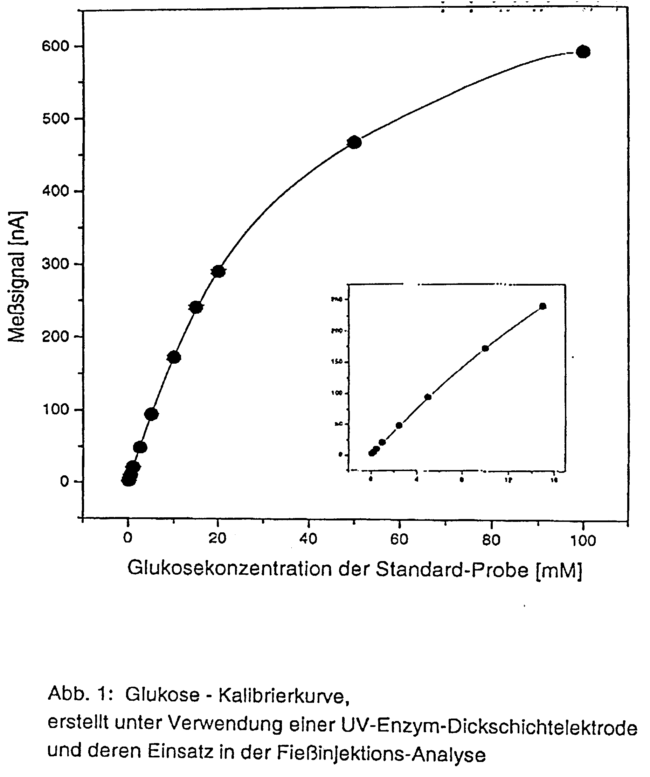

Nach Abschluß dieser Arbeiten ist die Enzymschicht vollkommen trocken, und der Sensor kann sofort zur Detektion des Analyten eingesetzt werden (**Abb. 1**). Die Ergebnisse zeigen die Abhängigkeit des gemessenen Signals in nA von der Konzentration der Glucose.

Der lineare Meßbereich kann an die jeweilige Applikation des Dickschicht-Biosensors (Bioprozeßkontrolle, medizinische Analytik) durch die Wahl eines geeigneten, die Porosität der Enzymschicht verändernden Materials (z.B. auf SiO₂ basierendes Mattierungsmittel aus dem Siebdruckbedarf) adaptiert werden.

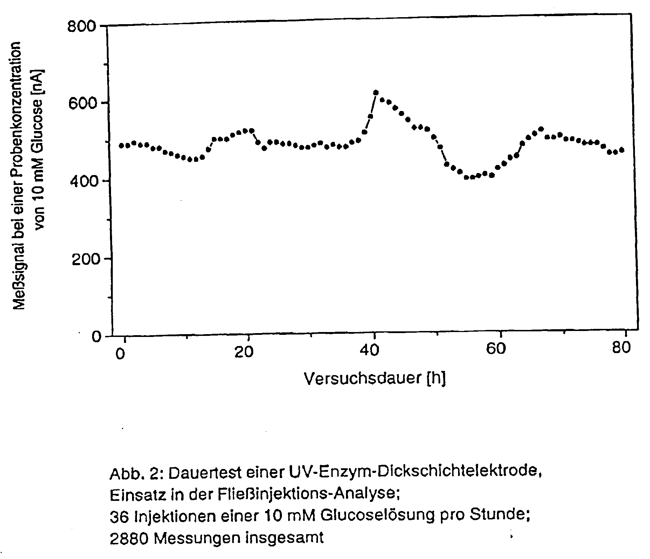

Der Dauereinsatz einer solchen UV-Enzym-Dickschichtelektrode über 80 Stunden in einem Fließinjektions-Analyse-System bei ständigem Spülen mit üblichen Puffer und einer Messung pro 100 sec (**Abb. 2**) zeigt die Langzeitstabilität der gedruckten und auspolymerisierten Enzymschicht auf der Elektrodenoberfläche. Aufgrund der Temperturabhängigkeit der Enzymreaktion treten im nicht thermostatisierten System tageszeitabhängige Signalschwankungen auf, wobei sich das durchschnittliche Sensorsignal eines Meßtages jedoch nicht verringert. Die durchschnittliche Standardabweichung von 10 aufeinanderfolgenden Meßsignalen liegt bei unter 1 %.

Die Meßergebnisse der in den Abbildungen gezeigten Kurven belegen die ausgezeichnete Arbeitsweise der erfindungsgemäßen Dickschicht-Biosensoren, die in Zusammenhang mit der amperometrischen Meßmethode eine hervorragende Meßgenauigkeit ergeben.

## Patentansprüche

1. UV-polymerisierbare Enzympaste zur Herstellung von Dickschicht-Biosensoren enthaltend
a) eine UV-polymerisierbare Siebdruckfarbe als Basismaterial,
b) mindestens ein Enzym, wobei das Enzym in das Basismaterial eingebracht ist und gegebenenfalls
c) Mediatoren und/oder Co-Faktoren.

2. UV-polymerisierbare Enzympaste nach Anspruch 1, wobei das Enzym in dem Basismaterial gelöst ist.

3. UV-polymerisierbare Enzympaste nach Anspruch 1, wobei das Enzym auf einen Träger immobilisiert in das Basismaterial eingebracht ist.

4. UV-polymerisierbare Enzympaste nach einem der Ansprüche 1 bis 3, wobei das Enzym eine Oxidase oder Dehydrogenase ist.

5. UV-polymerisierbare Enzympaste nach Anspruch 4, wobei das Enzym Glucoseoxidase ist.

6. UV-polymerisierbare Enzympaste nach Anspruch 4, wobei das Enzym Alkohol-Dehydrogenase oder Glucose-Dehydrogenase ist.

7. UV-polymerisierbare Enzympaste nach einem der Ansprüche 1 bis 6, wobei das Basismaterial ein Polymeres als Zusatz oder einen Zusatz auf Basis von SiO₂ enthält.

8. UV-polymerisierbare Enzympaste nach einem der Ansprüche 1 bis 6, wobei das Basismaterial ein auf SiO₂ basierendes Mattierungsmittel enthält.

9. Dickschicht-Biosensor, *gekennzeichnet* durch eine auspolymerisierte UV-polymerisierbare Enzympaste nach einem der Ansprüche 1 bis 8.

10. Biosensor nach Anspruch 9, wobei die UV-polymerisierbare Enzympaste zu einer Enzymschicht auspolymerisiert ist.

11. Biosensor nach Anspruch 9 oder 10, wobei die zu einer Enzymschicht auspolymerisierte Enzympaste eine Membran trägt.

12. Biosensor nach einem der Ansprüche 9 bis 11, wobei die Membran aus demselben Basismaterial mit demselben fakultativen Zusätzen wie die UV-polymerisierbare Enzympaste besteht, jedoch ohne Gehalt an Enzym.

## Claims

1. UV-polymerisable enzyme paste for the production of thick-layer biosensors, comprising
a) a UV-polymerisable screen printing ink as base material,
b) at least one enzyme, introduced into the base material, and, optionally,
c) mediators and/or co-factors.

2. UV-polymerisable enzyme paste according to claim 1, wherein the enzyme is dissolved in the base material.

3. UV-polymerisable enzyme paste according to claim 1, wherein the enzyme introduced into the base material has been immobilised on a support.

4. UV-polymerisable enzyme paste according to any one of claims 1 to 3, wherein the enzyme is an oxidase or dehydrogenase.

5. UV-polymerisable enzyme paste according to claim 4, wherein the enzyme is glucose oxidase.

6. UV-polymerisable enzyme paste according to claim 4, wherein the enzyme is alcohol dehydrogenase or glucose dehydrogenase.

7. UV-polymerisable enzyme paste according to any one of claims 1 to 6, wherein the base material comprises a polymer as additive or an SiO₂-based additive.

8. UV-polymerisable enzyme paste according to any one of claims 1 to 6, wherein the base material comprises a matting agent based on SiO₂.

9. Thick-layer biosensor, *characterised* by a polymerised UV-polymerisable enzyme paste according to any one of claims 1 to 8.

10. Biosensor according to claim 9, wherein the UV-polymerisable enzyme paste has been polymerised to form an enzyme layer.

11. Biosensor according to claim 9 or claim 10, wherein the enzyme paste polymerised to form an enzyme layer bears a membrane.

12. Biosensor according to any one of claims 9 to 11, wherein the membrane consists of the same base material, together with the same optional additives, as the UV-polymerisable enzyme paste but has no enzyme content.

## Revendications

1. Pâte enzymatique polymérisable par rayons ultraviolets pour la fabrication de biocapteurs à couche épaisse, contenant
a) une encre sérigraphique polymérisable par rayons ultraviolets, faisant fonction de matière de base,
b) au moins une enzyme, l'enzyme étant incorporée à la matière de base et, le cas échéant,
c) des médiateurs et/ou des cofacteurs.

2. Pâte enzymatique polymérisable par rayons ultraviolets selon la revendication 1, dans laquelle l'enzyme est dissoute dans la matière de base.

3. Pâte enzymatique polymérisable par rayons ultraviolets selon la revendication 1, dans laquelle l'enzyme, immobilisée sur un support, est incorporée à la matière de base.

4. Pâte enzymatique polymérisable par rayons ultraviolets selon l'une des revendications 1 à 3, dans laquelle l'enzyme est une oxydase ou une déhydrogénase.

5. Pâte enzymatique polymérisable par rayons ultraviolets selon la revendication 4, dans laquelle l'enzyme est une glucose-oxydase.

6. Pâte enzymatique polymérisable par rayons ultraviolets selon la revendication 4, dans laquelle l'enzyme est une alcool-déhydrogénase ou une glucose-déhydrogénase.

7. Pâte enzymatique polymérisable par rayons ultraviolets selon l'une des revendications 1 à 6, dans laquelle la matière de base contient un polymère employé comme additif ou un additif à base de SiO₂.

8. Pâte enzymatique polymérisable par rayons ultraviolets selon l'une des revendications 1 à 6, dans laquelle la matière de base contient un agent de matage à base de SiO₂.

9. Biocapteur à couche épaisse, caractérisé par une pâte enzymatique polymérisable par rayons ultraviolets polymérisée selon l'une des revendications 1 à 8.

10. Biocapteur selon la revendication 9, dans lequel la pâte enzymatique polymérisable par rayons ultraviolets est polymérisée sous forme de couche enzymatique.

11. Biocapteur selon la revendication 9 ou 10, dans lequel la pâte enzymatique polymérisée sous forme de couche enzymatique porte une membrane.

12. Biocapteur selon l'une des revendications 9 à 11, dans lequel la membrane est constituée de la même matière de base avec les mêmes additifs facultatifs que peut contenir la pâte enzymatique polymérisable par rayons ultraviolets, mais est dépourvue d'enzyme.
